# EUROPEAN PATENT APPLICATION

(11) **EP 4 224 481 A1**
(43) Date of publication of application: **09.08.2023**
(21) Application number: 21873848.2
(22) Date of filing: 23.04.2021
(51) Int. Cl.: G16H 20/10

(54) **CURRENCY TRANSACTION METHOD, SYSTEM AND APPARATUS BASED ON BLOCKCHAIN**

(30) Priority: 29.09.2020 CN 202011051076
(71) Applicant: BEIJING JINGDONG TUOXIAN TECHNOLOGY CO., LTD., Beijing 100176 (CN)
(72) Inventor: HAN, Lei, Beijing 100176 (CN); YANG, Jun, Beijing 100176 (CN); HE, Zhongqiang, Beijing 100176 (CN); GUAN, Luhui, Beijing 100176 (CN); XU, Shanbao, Beijing 100176 (CN)
(74) Representative: Bandpay & Greuter
(86) International application number: PCT/CN2021/089137
(87) International publication number: WO 2022/068184

(57) **Abstract**

Provided are a current transaction method, system and apparatus based on a blockchain. The solution is: receiving various service requests related to medication data of different users, and acquiring a first currency corresponding to the service requests of different users, wherein the first currency is obtained on the basis of performing medication rationality analysis on the medication data of the users; according to the first currency corresponding to the service requests of different users, determining a second currency in a blockchain reward pool corresponding to the first currency; and by means of an intelligent contract mechanism, completing a transfer of the second currency and completing a transaction, wherein the transfer is used for representing that the second currency is issued to each user in a reward manner, and the aim of the transaction is to exchange articles or services. By means of the solution, medication data of a user is managed by means of an intelligent contract of a blockchain, thereby implementing a method for managing the medication of a user by means of an intelligent contract of a blockchain.

## Description

### CROSS-REFERENCE TO RELATED APPLICATIONS

This patent application claims priority to Chinese Patent Application No. 202011051076.9 filed on September 29, 2020 by Beijing Jingdong Tuoxian Technology Co., Ltd. and entitled "METHOD, SYSTEM AND APPARATUS FOR MONEY TRANSACTION BASED ON BLOCKCHAIN", which is incorporated herein by reference.

### TECHNICAL FIELD

Embodiments of the present disclosure relate to the field of computer technology, in particular to the technical field of blockchain, and more particularly, to a method, system and apparatus for money transaction based on a blockchain.

### BACKGROUND

Medication compliance degree refers to the consistency of a patient's medication usage with the doctor's advice, and from the perspective of medication therapy, medication compliance degree refers to the degree to which the patient implements a medication treatment plan. There are many factors affecting medication compliance degree, involving patients, medical staff, society, family and other aspects, and poor medication compliance degree is a common phenomenon in elderly patients. With the decline of daily living ability and cognitive inadequacy in elderly patients, they are becoming more and more relying on medication. In addition, there are many types of medications that the elderly patients take, and the medication treatment plans are more complicated, so a medication-specific management approach is needed to improve a patient's medication compliance degree.

### SUMMARY

The present disclosure provides a method, system, and apparatus for money transaction based on a blockchain, a device, and a storage medium.

According to a first aspect of the present disclosure, a method for money transaction based on a blockchain is provided, the method including: receiving various service requests related to medication data of different users, and acquiring a first money corresponding to the service requests of different users, where the first money is obtained on the basis of performing medication rationality analysis on the medication data of the users; determining a second money in a blockchain reward pool corresponding to the first money, according to the first money corresponding to the service requests of different users; and completing a transfer of the second money and completing a transaction by means of a smart contract mechanism, where the transfer is used for representing that the second money is issued to each user in a reward method, and an aim of the transaction is to exchange articles or services.

In some embodiments, the receiving types of service requests related to medication usage data of different users, and acquiring first money corresponding to the types of service requests of the different users, comprises: receiving service purchase requests related to the medication usage data sent by different requesting nodes, and acquiring first money corresponding to the service purchase requests; receiving transaction requests related to the medication usage data between the nodes, and acquiring first money corresponding to the transaction requests, wherein the first money corresponding to the transaction requests represents a handling fee generated when a local node performs a money transaction with another node; and receiving data management requests related to the medication usage data sent by the different requesting nodes, and acquiring first money corresponding to the data management requests.

In some embodiments, the determining second money in a blockchain reward pool corresponding to the first money, according to the first money corresponding to the types of service requests of the different users, comprises: analyzing the first money of all users corresponding to the types of service requests, to obtain the second money in the blockchain reward pool corresponding to the first money; and/or analyzing the first money corresponding to all service requests of the users, to obtain the second money in the blockchain reward pool corresponding to the first money.

In some embodiments, a manner for transferring the second money comprises at least one of: investing the second money in a challenge competition, issuing the second money as a ranking reward, issuing the second money as a salary of a management node, and issuing the second money as a transaction fee to another node other than a local node participating in the transaction, wherein the management node represents a node that manages the medication usage data of different users.

In some embodiments, the method further comprises: monitoring the first money corresponding to the types of service requests of different users and the second money; and optimizing system parameters based on a result of the monitoring.

According to a second aspect of the present disclosure, a system for money transaction based on a blockchain is provided, the system includes: a service platform, wherein the service platform is configured to perform the above described method for money transaction based on a blockchain.

In some embodiments, the system further comprises: a requesting node; the requesting node is configured to receive medication usage data sent by a user, and use a money determination manner to generate first money corresponding to the medication usage data of the user, wherein the money determination manner is used to convert, based on medication compliance degree, the medication usage data sent by the user to the first money corresponding to the medication usage data of the user; and sending, according to the first money, types of service requests related to the medication usage data.

In some embodiments, the requesting node is further configured to record the medication usage data in the blockchain, and manage the recorded medication usage data.

According to a third aspect of the present disclosure, an apparatus for money transaction based on a blockchain is provided. The apparatus includes: an acquisition unit, configured to receive types of service requests related to medication usage data of different users, and acquire first money corresponding to the types of service requests of the different users, wherein the first money is obtained by performing medication rationality analysis on the medication usage data of the users; a determination unit, configured to determine second money in a blockchain reward pool corresponding to the first money, according to the first money corresponding to the types of service requests of the different users; and a transferring unit, configured to complete a transfer of the second money and complete a transaction by means of a smart contract mechanism, wherein the transfer is used for representing that the second money is issued to the users in a reward manner, and an aim of the transaction is in exchange for an article or service.

In some embodiments, the acquisition unit comprises: a first acquisition module, configured to receive service purchase requests related to the medication usage data sent by different requesting nodes, and acquire first money corresponding to the service purchase requests; a second acquisition module, configured to receive transaction requests related to the medication usage data between the nodes, and acquire first money corresponding to the transaction requests, wherein the first money corresponding to the transaction requests represents a handling fee generated when a local node performs money transaction with another node; and a third acquisition module, configured to receive data management requests related to the medication usage data sent by the different requesting nodes, and acquire first money corresponding to the data management requests.

In some embodiments, the determination unit comprises: a first analyzing module, configured to analyze the first money of all users corresponding to the types of service requests, to obtain the second money in the blockchain reward pool corresponding to the first money; and/or a second analyzing module, configured to analyze the first money corresponding to all service requests of the users, to obtain the second money in the blockchain reward pool corresponding to the first money.

In some embodiments, a manner for transferring the second money in the transferring unit comprises at least one of: investing the second money in a challenge competition, issuing the second money as a ranking reward, issuing the second money as a salary of a management node and issuing the second money as a transaction fee to another node other than a local node participating in the transaction, wherein the management node represents a node that manages the medication usage data of different users.

In some embodiments, the apparatus further comprises: a monitoring unit, configured to monitor the first money corresponding to the types of service requests of different users and the second money; and an optimizing unit, configured to optimize system parameters based on a result of the monitoring.

According to a fourth aspect of the present disclosure, an electronic device is provided. The electronic device includes: at least one processor; and a memory communicatively connected to the at least one processor; where the memory stores instructions executable by the at least one processor, and the instructions, when executed by the at least one processor, cause the at least one processor to perform the method according to any one of the implementations described in the first aspect.

According to a fifth aspect of the present disclosure, the present disclosure provides a non-transitory computer readable storage medium storing computer instructions, wherein, the computer instructions are used to cause the computer to perform the method according to any one of the implementations described in the first aspect.

According to the technology of the present disclosure, the first money of a corresponding value is obtained based on the data generated by a user's medication usage on time, and the first money may be in exchange for article(s) or service(s) of equivalent value in subsequent applying; by means of a smart contract of a blockchain, the user's medication usage data (that is, a medication status of the user and a medication usage implementation status) is managed, a method for managing medication usage of a user by means of a smart contract of a blockchain is implemented, and the method is automatically performed and cannot be cheated; by means of the blockchain smart contract technology, the data are authentic and credible; through blockchain rewards, the user's medication usage data is collected and a corresponding value is generated, so that the user can better take medicine on time and according to the medication regime, thus improving the user's enthusiasm for taking/using medication on time, and making the user's medication compliance degree higher.

It should be understood that the content described in this section is not intended to identify key features or embodiments of the present disclosure, nor is it intended to limit the scope of the present disclosure. Other features of the present disclosure will become readily understood from the following description.

### BRIEF DESCRIPTION OF THE DRAWINGS

The accompanying drawings are used for better understanding of the present solution, and do not constitute a limitation to the present disclosure.
Fig. 1 is a schematic diagram of a method for money transaction based on a blockchain according to a first embodiment of the present disclosure;
Fig. 2 is a scene diagram in which the method for money transaction based on a blockchain according to an embodiment of the present disclosure can be implemented;
Fig. 3 is a schematic diagram of the method for money transaction based on a blockchain according to a second embodiment of the present disclosure;
Fig. 4 is a schematic structural diagram of a system for money transaction based on a blockchain according to an embodiment of the present disclosure;
Fig. 5 is a schematic structural diagram of an apparatus for money transaction based on a blockchain according to an embodiment of the present disclosure; and
Fig. 6 is a block diagram of an electronic device used to implement the method for money transaction based on a blockchain according to an embodiment of the present disclosure.

### DETAILED DESCRIPTION OF EMBODIMENTS

Example embodiments of the present disclosure are described below with reference to the accompanying drawings, where various details of the embodiments of the present disclosure are included to facilitate understanding, and should be considered merely as exemplary. Therefore, those of ordinary skills in the art should realize that various changes and modifications can be made to the embodiments described herein without departing from the scope and spirit of the present disclosure. Similarly, for clearness and conciseness, descriptions of well-known functions and structures are omitted in the following description.

It should be noted that embodiments in the present disclosure and the features in the embodiments may be combined with each other on a non-conflict basis. Embodiments of the present disclosure will be described below in detail with reference to the accompanying drawings.

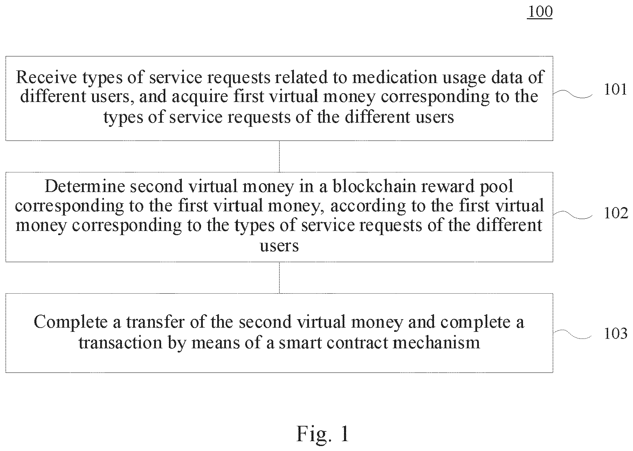

Fig. 1 shows a schematic diagram 100 of a method for money transaction based on a blockchain according to a first embodiment of the present disclosure. The method for money transaction based on a blockchain includes the following steps:
Step 101, receiving various types of service requests related to medication usage data of different users, and acquiring first money corresponding to the types of service requests of the different users.

In the present embodiment, an executing body (for example, a service platform) may receive various types of service requests related to the medication usage data of different users through wired connection or wireless connection, and acquire the first money corresponding to the types of service requests of the different users by analyzing content of the various types of service requests. The first money is used to represent a corresponding value obtained based on the medication usage data of the users, and the first money may be obtained on the basis of performing medication rationality analysis on the medication usage data of the users. The medication rationality is used to represent whether a user's medication usage is on time, according to the amount and in compliance with relevant requirements of the doctor's advice. The medication rationality analysis may refer to medication compliance. The higher the medication compliance degree of the user is, the more valuable the medication usage data of the corresponding user is, and the higher the first money is rewarded to the user. It should be noted that the above wireless connection may include, but is not limited to, 3G connection, 4G connection, 5G connection, WiFi connection, Bluetooth connection, WiMAX connection, Zigbee connection, UWB (ultra wideband) connection, and other wireless connection currently known or to be developed in the future.

Step 102, determining second money in a blockchain reward pool corresponding to the first money, according to the first money corresponding to the types of service requests of the different users.

In the present embodiment, the executing body may select from the first money corresponding to the types of service requests of the different users acquired in step 101, and determine, based on the result of the selecting, the second money in the blockchain reward pool corresponding to the first money. The result of the selecting may be part or all of the first money, the result of the selecting is used as the second money in the reward pool.

Step 103, completing a transfer of the second money and completing a transaction by means of a smart contract mechanism.

In the present embodiment, the executing body may complete the transfer of the second money and complete the transaction by means of the smart contract mechanism of the blockchain, according to the second money obtained in step 102. The transfer is used to represent issuing the second money to each user in a reward manner, and an aim of the transaction is in exchange for an article or service. A manner for transferring the second money may include: basic mining, inviting new user, authorizing data to other users, and participating in clinical trials, etc.

It should be noted that the smart contract mechanism of the blockchain is a well-known technology that is widely researched and applied, and detailed description thereof will be omitted.

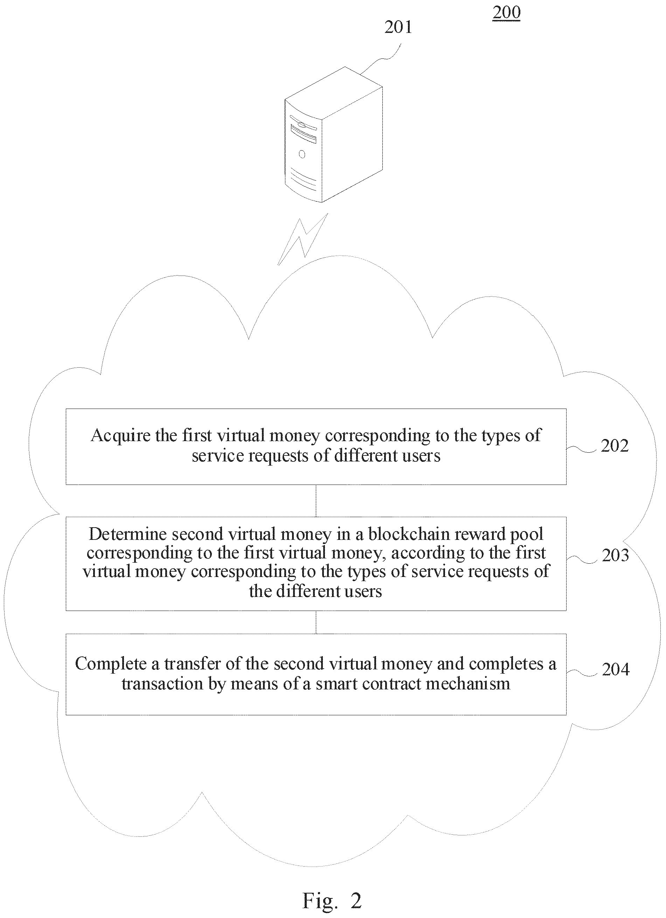

With further reference to Fig. 2, a method 200 for money transaction based on a blockchain in the present embodiment runs in a service platform 201. When receiving various types of service requests related to medication usage data of different users, the service platform 201 acquires first money corresponding to the types of service requests of the different users 202, then the service platform 201 determines second money in a blockchain reward pool corresponding to the first money, according to the first money corresponding to the types of service requests of the different users 203, and finally the service platform 201 completes a transfer of the second money and completes a transaction by means of a smart contract mechanism 204.

In the method for money transaction based on a blockchain provided by the above embodiment of the present disclosure, based on the data generated by a user's medication usage on time, the first money of a corresponding value is obtained, and the first money may be in exchange for article(s) or service(s) of equivalent value in subsequent applying; by means of a smart contract of a blockchain, the user's medication usage data (that is, a medication status of the user and an medication usage implementation status) is managed, a method for managing medication usage of a user by means of a smart contract of a blockchain is implemented, and the method is automatically performed and cannot be cheated; by means of the blockchain smart contract technology, the data is authentic and credible; through blockchain rewards, the user's medication usage data is collected and a corresponding value is generated, so that the user can better take/use medicine according to the medication regime, thus improving the user's enthusiasm for taking medication on time, and making the user's medication compliance degree higher.

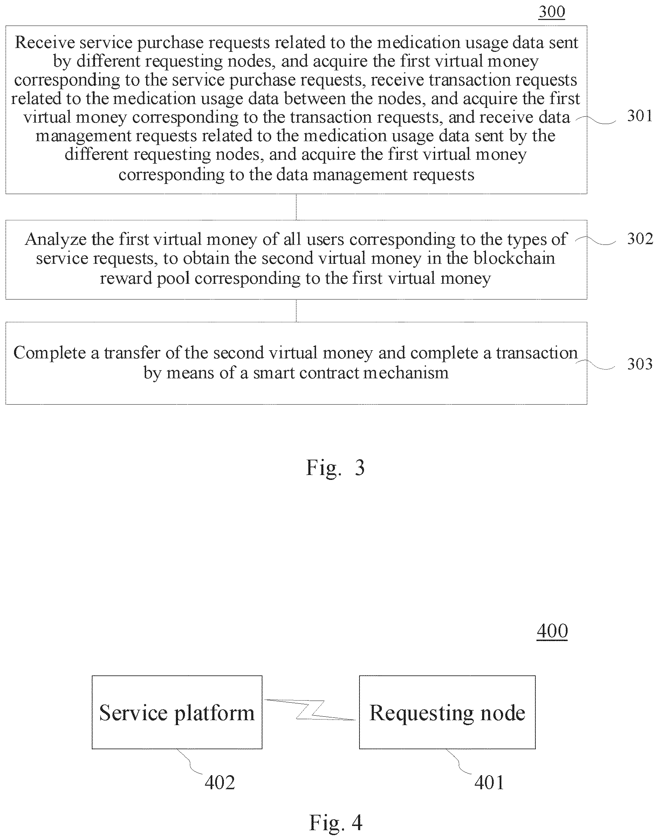

With further reference to Fig. 3, illustrating a schematic diagram 300 of a second embodiment of the method for money transaction based on a blockchain. The flow of the method includes the following steps:

Step 301, receiving service purchase requests related to the medication usage data sent by different requesting nodes, and acquiring first money corresponding to the service purchase requests, receiving transaction requests related to the medication usage data between the nodes, and acquiring first money corresponding to the transaction requests, and receiving data management requests related to the medication usage data sent by the different requesting nodes, and acquiring first money corresponding to the data management requests.

In the present embodiment, the executing body may receive the service purchase requests related to the medication usage data sent by the different requesting nodes, receive the transaction requests related to the medication usage data between the nodes, and receive the data management requests related to the medication usage data sent by the different requesting nodes, and then acquire the corresponding first money according to the received different types of requests. The first money is obtained on the basis of performing medication rationality analysis on the medication usage data of the users, the first money corresponding to the transaction requests may represent a handling fee generated when a local node performs a money transaction with another node, the first money corresponding to the service purchase requests may represent a first money that the user needs to pay for purchasing health services, and the first money corresponding to the data management requests may represent a management fee required for management operations such as query and change of data related to the medication usage data.

Step 302, analyzing the first money of all users corresponding to the various types of service requests, to obtain the second money in the blockchain reward pool corresponding to the first money.

In the present embodiment, the executing body may analyze the first money of all the users corresponding to the various types of service requests acquired in step 301, to obtain the second money in the blockchain reward pool corresponding to the first money.

In some alternative implementations of the present embodiment, the determining a second money in a blockchain reward pool corresponding to the first money, further includes: analyzing the first money corresponding to all service requests of the users, to obtain the second money in the blockchain reward pool corresponding to the first money. Obtaining the second money from different dimensions expands an application arrange of the data.

Step 303, completing a transfer of the second money and completing a transaction by means of a smart contract mechanism.

In some alternative implementations of the present embodiment, a method for transferring the second money includes at least one of: investing the second money in a challenge competition, issuing the second money as a ranking reward, issuing the second money as a salary of a management node and issuing the second money as a transaction fee to another node (third-party node) other than a local node participating in the transaction, where the management node represents a node that manages the medication usage data of different users. The data generated by users' rational medication usage may eventually be transformed into the needs of the third party/parties, thereby obtaining corresponding values; users' medication compliance degree may be improved by obtaining rewards in various ways, and users with high compliance degree may acquire more value by maintaining this good habit, thus forming a virtuous circle and improving medication compliance degree.

In some alternative implementations of the present embodiment, the method further includes: monitoring the first money and the second money corresponding to the various service requests of different users; and optimizing system parameters based on a monitoring result. The optimization takes keeping the value of the first money as an adjustment target. By taking medication on time, the user may make the first money reach balance between income and outlay, and prevent the first money from depreciating due to inflation in the system, so as to reduce a risk in medication compliance of users and achieve effective management.

In the present embodiment, the operation of step 303 is basically the same as the operation of step 103 in the embodiment shown in Fig. 1, and detailed description thereof will be omitted.

As can be seen from Fig. 3, compared with the embodiment corresponding to Fig. 1, the schematic diagram 300 of the method for money transaction based on a blockchain in the present embodiment receives the service purchase requests related to the medication usage data sent by the different requesting nodes, and acquires the first money corresponding to the service purchase requests, receives the transaction requests related to the medication usage data between the nodes, and acquires the first money corresponding to the transaction requests, receives the data management requests related to the medication usage data sent by the different requesting nodes, and acquires the first money corresponding to the data management requests, analyzes the first money of all the users corresponding to the various service requests, to obtain the second money in the blockchain reward pool corresponding to the first money, and completes the transfer of the second money and completes the transaction by means of the smart contract mechanism. By acquiring the first money corresponding to the various types of service requests, a range of data collection is expanded, and the data is more refined and richer, thereby implementing a more refined method for managing medication usage of a user by means of a smart contract of a blockchain.

With further reference to Fig. 4, the present disclosure provides a system for money transaction based on a blockchain, as shown in Fig. 4, the system includes: a requesting node 401 and a service platform 402, where the service platform is configured to perform the method for money transaction based on a blockchain. The requesting node is configured to receive medication usage data sent by a user, and use a method for determining a money to generate a first money corresponding to the medication usage data of the user, where the method for determining a money is used to represent converting the medication usage data to the first money based on medication compliance degree; and sending various types of service requests related to the medication usage data, according to the first money. The higher the medication compliance degree of the user is, the more valuable the medication usage data of the corresponding user is, and the higher a first money reward the user obtains.

In the system, the requesting node is further configured to record the medication usage data in the blockchain, and manage the recorded medication usage data. By recording the data on the blockchain, the data can be authentic and unchangeable, at the same time the data may be authorized and managed to generate a value.

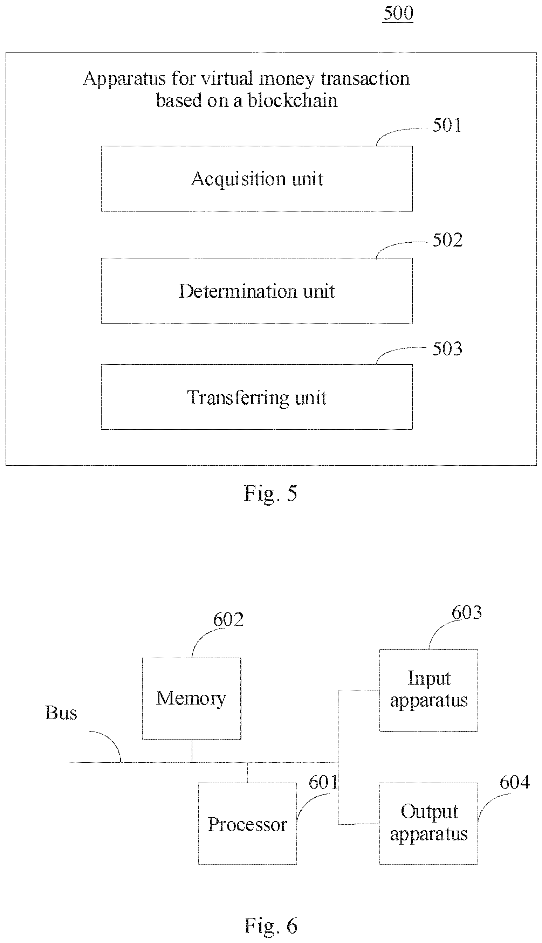

With further reference to Fig. 5, as an implementation of the method shown in the above Figs. 1-3, an embodiment of the present disclosure provides an apparatus for money transaction based on a blockchain. The embodiment of the apparatus corresponds to the embodiment of the method shown in Fig. 1. Particularly, the apparatus may be used in various electronic device.

As shown in Fig. 5, an apparatus 500 for money transaction based on a blockchain in the present embodiment includes: an acquisition unit 501, a determination unit 502 and a transferring unit 503. The acquisition unit is configured to receive various types of service requests related to medication usage data of different users, and acquire a first money corresponding to the types of service requests of the different users, where the first money is obtained by performing medication rationality analysis on the medication usage data of the users. The determination unit is configured to determine second money in a blockchain reward pool corresponding to the first money, according to the first money corresponding to the types of service requests of the different users. The transferring unit is configured to complete a transfer of the second money and complete a transaction by means of a smart contract mechanism, wherein the transfer is used for representing that the second money is issued to the users in a reward manner, and an aim of the transaction is in exchange for an article or service.

In the present embodiment, in the apparatus 500 for money transaction based on a blockchain: for the detailed processing and the technical effects of the acquisition unit 501, the determination unit 502 and the transferring unit 503, reference may be made to the relevant descriptions of step 101 to step 103 in the corresponding embodiment of Fig. 1, respectively, and detailed description thereof will be omitted.

In some alternative implementations of the present embodiment, the acquisition unit includes: a first acquisition module, configured to receive service purchase requests related to the medication usage data sent by different requesting nodes, and acquire first money corresponding to the service purchase requests; a second acquisition module, configured to receive transaction requests related to the medication usage data between the nodes, and acquire first money corresponding to the transaction requests, wherein the first money corresponding to the transaction requests represents a handling fee generated when a local node performs money transaction with another node; and a third acquisition module, configured to receive data management requests related to the medication usage data sent by the different requesting nodes, and acquire first money corresponding to the data management requests.

In some alternative implementations of the present embodiment, the determination unit includes: a first analyzing module, configured to analyze the first money of all users corresponding to the types of service requests, to obtain the second money in the blockchain reward pool corresponding to the first money; and/or a second analyzing module, configured to analyze the first money corresponding to all service requests of the users, to obtain the second money in the blockchain reward pool corresponding to the first money.

In some alternative implementations of the present embodiment, a manner for transferring the second money in the transferring unit includes at least one of: investing the second money in a challenge competition, issuing the second money as a ranking reward, issuing the second money as a salary of a management node and issuing the second money as a transaction fee to another node other than a local node participating in the transaction, wherein the management node represents a node that manages the medication usage data of different users.

In some alternative implementations of the present embodiment, the apparatus further includes: a monitoring unit, configured to monitor the first money corresponding to the types of service requests of different users and the second money; and an optimizing unit, configured to optimize system parameters based on a result of the monitoring.

According to an embodiment of the present disclosure, the present disclosure also provides an electronic device and a readable storage medium.

As shown in Fig. 6, is a block diagram of an electronic device of the method for money transaction based on a blockchain according to an embodiment of the present disclosure. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processors, cellular phones, smart phones, wearable devices, and other similar computing apparatuses. The components shown herein, their connections and relationships, and their functions are merely examples, and are not intended to limit the implementation of the present disclosure described and/or claimed herein.

As shown in Fig. 6, the electronic device includes: one or more processors 601, a memory 602, and interfaces for connecting various components, including high-speed interfaces and low-speed interfaces. The various components are interconnected using different buses and may be mounted on a common motherboard or otherwise as desired. The processor may process instructions executed within the electronic device, including instructions stored in or on the memory to display graphical information of a GUI on an external input/output apparatus, such as a display device coupled to the interface. In other embodiments, a plurality of processors and/or a plurality of buses may be used with a plurality of memories and a plurality of memories, if desired. Likewise, a plurality of electronic devices may be connected, each providing some of the necessary operations (e.g., as a server array, a set of blade servers, or a multiprocessor system). One processor 601 is used as an example in Fig. 6.

The memory 602 is a non-transitory computer readable storage medium provided by the present disclosure. The memory stores instructions executable by at least one processor, so that the at least one processor executes the method for money transaction based on a blockchain provided by the present disclosure. The non-transitory computer readable storage medium of the present disclosure stores computer instructions, and the computer instructions are used to cause the computer to perform the method for money transaction based on a blockchain provided by the present disclosure.

As a non-transitory computer readable storage medium, the memory 602 may be used to store non-transitory software programs, non-transitory computer-executable programs, and modules, such as program instructions/modules (for example, the acquisition unit 501, the determination unit 502 and the transferring unit 503 shown in Fig. 5) corresponding to the method for money transaction based on a blockchain in embodiments of the present disclosure. The processor 601 executes various functional applications and data processing of the server by running the non-transitory software programs, instructions and modules stored in the memory 602, that is, implements the method for money transaction based on a blockchain in the above method embodiments.

The memory 602 may include a stored program area and a stored data area, where the stored program area may store an operating system, an application program required by at least one function; and the stored data area may store data created according to the use of the electronic device for money transaction based on a blockchain, etc. Additionally, the memory 602 may include a high-speed random access memory, and may also include a non-transitory memory, such as at least one magnetic disk storage device, a flash memory device, or other non-transitory solid-state storage device. In some embodiments, the memory 602 may optionally include memories located remotely from the processor 601, and these remote memories may be connected to the electronic device for money transaction based on a blockchain via a network. Examples of such network include, but are not limited to, the Internet, an intranet, a local area network, a mobile communication network, and combinations thereof.

The electronic device of the method for money transaction based on a blockchain may further include: an input apparatus 603 and an output apparatus 604. The processor 601, the memory 602, the input apparatus 603 and the output apparatus 604 may be connected via a bus or in other ways, and connection via a bus is used as an example in Fig. 6.

The input apparatus 603 may receive input digital or character information, and generate key signal inputs related to user settings and function control of the electronic device of the method for money transaction based on a blockchain, such as touch screen, keypad, mouse, trackpad, touchpad, pointing stick, one or more mouse buttons, trackball, joystick and other input apparatuses. The output apparatus 604 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive data and instructions from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide machine instructions and/or data to the programmable processor, including machine readable medium that receives machine instructions as machine readable signals. The term "machine readable signal" refers to any signal used to provide machine instructions and/or data to the programmable processor.

In order to provide interaction with a user, the systems and technologies described herein may be implemented on a computer, the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network). Examples of the communication network include: local area networks (LAN), wide area networks (WAN), the Internet, and blockchain networks.

The computer system may include a client and a server. The client and the server are generally far from each other and usually interact through the communication network. The relationship between the client and the server is generated by computer programs that run on the corresponding computer and have a client-server relationship with each other.

According to the technical solution of embodiments of the present disclosure, the first money of a corresponding value may be obtained based on the data generated by a user's medication usage on time, and the first money may be in exchange for article(s) or service(s) of equivalent value in subsequent applying; by means of a smart contract of a blockchain, the user's medication usage data (that is, a medication status of the user and a medication usage implementation status) is managed, a method for managing medication usage of a user by means of a smart contract of a blockchain is implemented, and the method is automatically performed and cannot be cheated; by means of the blockchain smart contract technology, the data is authentic and credible; through blockchain rewards, the user's medication usage data is collected and a corresponding value is generated, so that the user can better take/use medicine on time and according to the medication regime, thus improving the user's enthusiasm for taking medication on time, and making the user's medication compliance degree higher.

It should be understood that the various forms of processes shown above may be used to reorder, add, or delete steps. For example, the steps described in the present disclosure may be performed in parallel, sequentially, or in different orders. As long as the desired results of the technical solution disclosed in the present disclosure can be achieved, no limitation is made herein.

The above specific embodiments do not constitute limitation on the protection scope of the present disclosure. Those skilled in the art should understand that various modifications, combinations, sub-combinations and substitutions may be made according to design requirements and other factors. Any modification, equivalent replacement and improvement made within the spirit and principle of the present disclosure shall be included in the protection scope of the present disclosure.

## Claims

1. A method for money transaction based on a blockchain, the method comprising:
receiving types of service requests related to medication usage data of different users, and acquiring first money corresponding to the types of service requests of the different users, wherein the first money is obtained by performing medication rationality analysis on the medication usage data of the users;
determining second money in a blockchain reward pool corresponding to the first money, according to the first money corresponding to the types of service requests of the different users; and
completing a transfer of the second money and completing a transaction by means of a smart contract mechanism, wherein the transfer is used for representing that the second money is issued to the users in a reward manner, and an aim of the transaction is in exchange for an article or a service.

2. The method according to claim 1, wherein the receiving types of service requests related to medication usage data of different users, and acquiring first money corresponding to the types of service requests of the different users, comprises:
receiving service purchase requests related to the medication usage data sent by different requesting nodes, and acquiring first money corresponding to the service purchase requests;
receiving transaction requests related to the medication usage data between the nodes, and acquiring first money corresponding to the transaction requests, wherein the first money corresponding to the transaction requests represents a handling fee generated when a local node performs a money transaction with another node; and
receiving data management requests related to the medication usage data sent by the different requesting nodes, and acquiring first money corresponding to the data management requests.

3. The method according to claim 1, wherein the determining second money in a blockchain reward pool corresponding to the first money, according to the first money corresponding to the types of service requests of the different users, comprises:
analyzing the first money of all users corresponding to the types of service requests, to obtain the second money in the blockchain reward pool corresponding to the first money; and/or
analyzing the first money corresponding to all service requests of the users, to obtain the second money in the blockchain reward pool corresponding to the first money.

4. The method according to claim 1, wherein a manner for transferring the second money comprises at least one of: investing the second money in a challenge competition, issuing the second money as a ranking reward, issuing the second money as a salary of a management node, and issuing the second money as a transaction fee to another node other than a local node participating in the transaction, wherein the management node represents a node that manages the medication usage data of different users.

5. The method according to claim 1, wherein the method further comprises:
monitoring the first money corresponding to the types of service requests of different users and the second money; and
optimizing system parameters based on a result of the monitoring.

6. A system for money transaction based on a blockchain, the system comprising: a service platform, wherein the service platform is configured to perform the method for money transaction based on a blockchain according to any one of claims 1-5.

7. The system according to claim 6, wherein the system further comprises: a requesting node; and
the requesting node is configured to receive medication usage data sent by a user, and use a money determination manner to generate first money corresponding to the medication usage data of the user, wherein the money determination manner is used to convert, based on medication compliance degree, the medication usage data sent by the user to the first money corresponding to the medication usage data of the user; and sending, according to the first money, types of service requests related to the medication usage data.

8. The system according to claim 6, wherein the requesting node is further configured to record the medication usage data in the blockchain, and manage the recorded medication usage data.

9. An apparatus for money transaction based on a blockchain, the apparatus comprising:
an acquisition unit, configured to receive types of service requests related to medication usage data of different users, and acquire first money corresponding to the types of service requests of the different users, wherein the first money is obtained by performing medication rationality analysis on the medication usage data of the users;
a determination unit, configured to determine second money in a blockchain reward pool corresponding to the first money, according to the first money corresponding to the types of service requests of the different users; and
a transferring unit, configured to complete a transfer of the second money and complete a transaction by means of a smart contract mechanism, wherein the transfer is used for representing that the second money is issued to the users in a reward manner, and an aim of the transaction is in exchange for an article or service.

10. The apparatus according to claim 9, wherein the acquisition unit comprises:
a first acquisition module, configured to receive service purchase requests related to the medication usage data sent by different requesting nodes, and acquire first money corresponding to the service purchase requests;
a second acquisition module, configured to receive transaction requests related to the medication usage data between the nodes, and acquire first money corresponding to the transaction requests, wherein the first money corresponding to the transaction requests represents a handling fee generated when a local node performs money transaction with another node; and
a third acquisition module, configured to receive data management requests related to the medication usage data sent by the different requesting nodes, and acquire first money corresponding to the data management requests.

11. The apparatus according to claim 8, wherein the determination unit comprises:
a first analyzing module, configured to analyze the first money of all users corresponding to the types of service requests, to obtain the second money in the blockchain reward pool corresponding to the first money; and/or
a second analyzing module, configured to analyze the first money corresponding to all service requests of the users, to obtain the second money in the blockchain reward pool corresponding to the first money.

12. The apparatus according to claim 9, wherein a manner for transferring the second money in the transferring unit comprises at least one of: investing the second money in a challenge competition, issuing the second money as a ranking reward, issuing the second money as a salary of a management node and issuing the second money as a transaction fee to another node other than a local node participating in the transaction, wherein the management node represents a node that manages the medication usage data of different users.

13. The apparatus according to claim 9, wherein the apparatus further comprises:
a monitoring unit, configured to monitor the first money corresponding to the types of service requests of different users and the second money; and
an optimizing unit, configured to optimize system parameters based on a result of the monitoring.

14. An electronic device, comprising:
at least one processor; and
a memory communicatively connected to the at least one processor; wherein,
the memory stores instructions executable by the at least one processor, and the instructions, when executed by the at least one processor, cause the at least one processor to perform the method according to any one of claims 1-5.

15. A non-transitory computer readable storage medium storing computer instructions, wherein, the computer instructions are used to cause the computer to perform the method according to any one of claims 1-5.
